# EUROPÄISCHE PATENTSCHRIFT

(11) **EP 3 377 782 B1**
(45) Veröffentlichungstag und Bekanntmachung des Hinweises auf die Patenterteilung: **06.05.2020**
(21) Anmeldenummer: 16797894.9
(22) Anmeldetag: 17.11.2016
(51) Int. Cl.: F16C 33/04, A61F 2/34, F16C 11/06, A61F 2/30

(54) **RINGFÖRMIGES KERAMISCHES INSERT FÜR DIE ENDOPROTHETIK**
ANNULAR CERAMIC INSERT FOR ENDOPROSTHETICS
INSERT CÉRAMIQUE ANNULAIRE POUR L'ENDOPROTHÈSE

(30) Priorität: 19.11.2015 DE 102015222818
(43) Veröffentlichungstag der Anmeldung: 26.09.2018
(73) Patentinhaber: CeramTec GmbH, 73207 Plochingen (DE)
(72) Erfinder: SCHNEIDER, Norbert, 73614 Schorndorf (DE)
(86) Internationale Anmeldenummer: PCT/EP2016/077941
(87) Internationale Veröffentlichungsnummer: WO 2017/085161

(56) Entgegenhaltungen:
- WO-A1-2011/135074
- DE-A1-102009 027 371
- US-A- 5 263 988
- US-A- 5 549 693
- US-A- 5 919 236
- US-A1- 2013 046 388
- US-B2- 8 801 797

## Beschreibung

Die Erfindung betrifft einen Gelenkpfannenersatz mit einem keramischen Insert für die Gleitpaarung in der Endoprothetik, wobei das Insert eine Außenseite und eine Innenseite aufweist und auf der Innenseite ein hemispärischer Gleitbereich zur Aufnahme eines kugelförmigen Gleitpartners ausgebildet ist.

Keramische Inserts für die Gleitpaarung in der Hüftendeprothetik sind hemisphärisch ausgebildet und überdecken ca. 50 % des Prothesenkopfs. Der Mittelpunkt der Gleitfläche liegt auf der Ebene der Stirnfläche oder leicht darüber oder darunter.

Das Insert hat auf der Außenseite am Äquator umlaufend eine mehr oder weniger breite Klemmfläche (konisch oder zylindrisch) mit der es in eine metallische Schale (Metallschale) eingesetzt wird (vormontiert oder intraoperativ).

Die übrige Rückseite bis zum Pol steht nicht im Kontakt zur Metallschale, muss aber aus Stabilitätsgründen eine Mindestwandstärke haben.

Die Lastübertragung zwischen Hüftkopf und Insert bzw. Hüftpfanne in der Gleitfläche ist bei dieser Paarung punkt- bzw. kreisflächenförmig, da zwischen Kugel- und Kalottendurchmesser eine positive Clearance vorliegt. Die Belastung wird dabei durch den Hüftkopf achsenparallel auf das Insert übertragen.

US5 549 693 A zeigt ein Implantat, dessen Gleitbereich im Innenraum zum Teil aus einem keramischen Ring besteht, der in eine halbschalige Zwischenschale eingesetzt ist. Diese Zwischenschale ermöglicht die bewegungsfreie Befestigung in einer Außenschale. US5 263 988 A zeigt ein Implantat dessen Gleitbereich aus einem Polyethylen-Insert besteht, das halbschalig ausgeführt ist und durch einen Verschlussring in der Außenschale befestigt ist. DE 10 2009 027371 A1 zeigt eine Bandscheibenendoprothese, deren Innenbereich über Radien in den Außenbereich übergeht. WO2011/135074 und US 5,919,236 zeigen halbschalige Inserts, die in Metallschalen eingebracht werden.

Der Erfindung liegt die Aufgabe zugrunde, einen Gelenkpfannenersatz mit einem keramischen Insert nach dem Oberbegriff des Anspruchs 1 so zu verbessern, dass die Bautiefe für Metallschale plus Insert verringert ist, so dass eine weniger tiefe Ausfräsung im Beckenknochen nötig ist. Außerdem sollen keine punktförmigen Lasten, sondern streifenförmige Lasten mit geringeren Maximalwerten, ähnlich der physiologischen Lastaufnahme vorhanden sein. Ferner soll sich ein Kostenvorteil durch Einsparung von Material und Nettovolumen bei der Herstellung (z.B. Nutzraum im Ofen) ergeben.

Erfindungsgemäß wird diese Aufgabe durch einen Gelenkpfannenersatz mit keramischem Insert nach den Merkmalen des Anspruchs 1 gelöst.

Dadurch, dass das Insert als Ring oder ringförmig ausgebildet ist, entsteht ein Bauteil, das innen einen Ringabschnitt einer Sphäre darstellt. Der hintere Abschluss wird bevorzugt über Radien dargestellt, die die Innenseite mit der Außenseite verbinden. Dadurch hat das Insert nur ca. die halbe Bautiefe, wie handelsübliche Inserts.

Als Vorteile ergeben sich:
- Geringere Bautiefe für Schale plus Insert, daher ist eine weniger tiefe Ausfräsung im Beckenknochen nötig.
- Keine punktförmige Lasten, sondern streifenförmige Lasten mit geringeren Maximalwerten, ähnlich der physiologischen Lastaufnahme
- Einsparung von Material und Nettovolumen bei der Herstellung (z.B. Nutzraum im Ofen), wodurch sich ein Kostenvorteil ergibt

In einer erfindungsgemäßen Ausgestaltung geht die Innenseite über Radien in die Außenseite über. Dies gilt sowohl für das obere als auch das untere Ende des Inserts. Es gibt somit keine scharfen Kanten, wodurch die Stabilität verbessert ist.

Bevorzugt bedeckt der hemispärische Gleitbereich die gesamte Innenseite, wodurch die gesamte Innenseite für die Gleitpaarung zur Verfügung steht. Dies verringert die Bautiefe auf ein Minnimum.

Auf der Außenseite ist zumindest in Teilbereichen eine Klemmfläche angeordnet, die konisch oder zylindrisch ausgebildet ist. Diese Klemmfläche dient zur Verankerung in einer äußeren Metallschale.

Die Klemmfläche auf der Außenseite des Inserts ist konisch oder zylindrisch ausgebildet, so dass das Insert fest mit der Metallschale verbindbar ist.

Bevorzugt hat das Insert eine Breite von 5-20 mm. Bei diesen Breiten wird wenig Bauraum benötigt und ist unerwartet die Klemmkraft trotzden ausreichend.

Das erfindungsgemäße keramische Insert wird als Ring ausgebildet, der bevorzugt an der Oberseite die gleiche Innenkontur, Stirnfläche und Klemmfläche wie herkömmliche Inserts ausweist, aber nur eine Breite von 5-20 mm hat.

In den Figuren werden vorteilhafte Ausführungsformen des erfindungsgemäßen keramischen Inserts beschrieben.

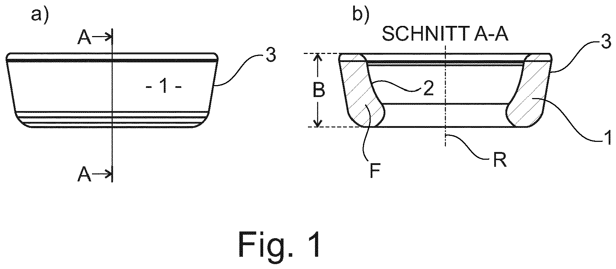

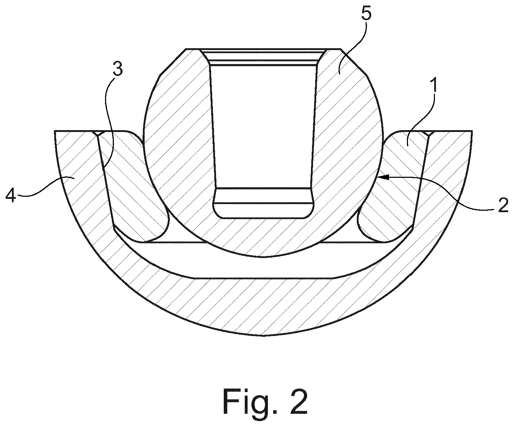

In den Figuren 1a und 1b ist ein erfindungsgemäßes ringförmiges keramisches Insert 1 gezeigt. Figur 1a zeigt dieses Insert 1 in einer Ansicht und Figur 1b im Schnitt entlang der Linie A-A gemäß Figur 1a. Das Insert 1 hat einen inneren Ringabschnitt einer Sphäre, auch als hemispärischer Gleitbereich 2 bezeichnet. Auf dieser artikuliert bei einer Hüftgelenkprothese der Prothesenkopf, siehe Figur 2. Auf der Außenseite des Inserts ist eine umlaufende Klemmfläche 3 angeordnet, mit der das Insert in einer Metallschale 4 verankert werden kann. Die Breite des Insert B liegt zwischen 5 bis 20 mm. Mit R ist die Rotationachse bezeichnet. Mit einer ringförmigen Ausbildung ist ein Insert verstanden, welches von der Rotationssymmetrie in Teilbereichen abweicht.
Figur 2 zeigt das erfindungsgemäße Insert 1, eingesetzt in eine Metallschale 4. In das Insert 1 ist ein Prothesenkopf 5 eingesetzt.

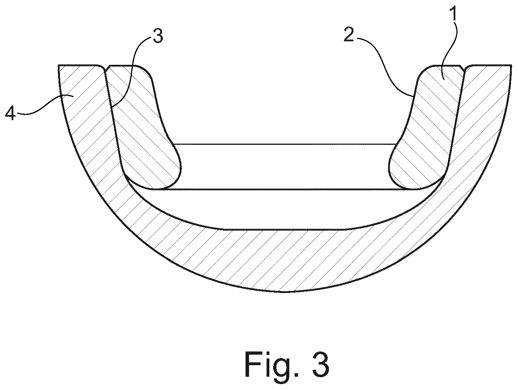

Figur 3 zeigt in einem Schnitt ein erfindungsgemäßes Insert 1 eingesetzt in eine Metallschale 4, mit einem inneren Ringabschnitt einer Sphäre bzw. einem hemispärischen Gleitbereich 2. Mit dem Bezugszeichen 3 ist eine bevorzugt umlaufende Klemmfläche bezeichnet. Es können auch Unterbrechungen in der Klemmfläche vorhanden sein.

Unter einem Ring wird in dieser Anmeldung ein Körper verstanden, der aus einer Fläche F (siehe Figur 1b) gebildet ist, die um eine Rotationsachse R rotiert. Unter der Breite B des Ringes wird dessen Erstreckung entlang der Rotationsachse R verstanden. Unter ringförmig wird ein Ring verstanden, der in Teilbereichen eine von der Rotationssymmetrie abweichende Ausbildung aufweist.

## Patentansprüche

1. Gelenkpfannenersatz für die Gleitpaarung in der Endoprothetik, bestehend aus einer Metallschale (4) und einem in die Metallschale (4) geklemmtem keramischen Insert (1), wobei das Insert (1) eine Außenseite und eine Innenseite aufweist und auf der Innenseite ein hemispärischer Gleitbereich (2) zur Aufnahme eines kugelförmigen Gleitpartners ausgebildet ist, wobei das Insert (1) als Ring oder ringförmig ausgebildet ist, und auf der Außenseite zumindest in Teilbereichen umlaufend eine konisch oder zylindrisch ausgebildete Klemmfläche (3) aufweist, so dass das Insert nur über die Klemmfläche mit der Metallschale verbunden ist.

2. Gelenkpfannenersatz nach Anspruch 1, **dadurch gekennzeichnet, dass** das Insert (1) eine Breite (B) von 5 bis 20 mm hat.

3. Gelenkpfannenersatz nach Anspruch 1 oder 2, **dadurch gekennzeichnet, dass** die Innenseite über Radien in die Außenseite übergeht.

4. Gelenkpfannenersatz nach einem der Ansprüche 1 bis 3, **dadurch gekennzeichnet, dass** der hemispärische Gleitbereich (2) die gesamte Innenseite bedeckt.

5. Gelenkpfannenersatz nach einem der vorstehenden Ansprüche, **dadurch gekennzeichnet, dass** die umlaufende Klemmfläche (3) Unterbrechungen aufweist.

6. Verwendung eines Gelenkpfannenersatz nach einem der Ansprüche 1 bis 5 in der Hüft-, Schulter-, Zehen- oder Knieendoprothetik.

## Claims

1. Joint socket replacement for sliding pairing in arthroplasty, consisting of a metal shell (4) and a ceramic insert (1) clamped into the metal shell (4), wherein the insert (1) has an outer face and an inner face and a hemispherical sliding region (2) is formed on the inner face to accommodate a spherical sliding partner, wherein the insert (1) is designed as a ring or is ring-shaped and has a conical or cylindrical clamping surface (3) on the outer face at least in portions, such that the insert is connected to the metal shell only via the clamping surface.

2. Joint socket replacement according to claim 1, **characterized in that** the insert (1) has a width (B) of from 5 to 20 mm.

3. Joint socket replacement according to either claim 1 or claim 2, **characterized in that** the inner face merges into the outer face via radii.

4. Joint socket replacement according to any of claims 1 to 3, **characterized in that** the hemispherical sliding region (2) covers the entire inner face.

5. Joint socket replacement according to any of the preceding claims, **characterized in that** the circumferential clamping surface (3) has interruptions.

6. Use of a joint socket replacement according to any of claims 1 to 5 in hip, shoulder, toe or knee arthroplasty.

## Revendications

1. Coque acétabulaire de remplacement pour l'appariement coulissant dans le cadre d'une arthroplastie, composée d'une enveloppe métallique (4) et d'un insert en céramique (1) serré dans l'enveloppe métallique (4), dans laquelle l'insert (1) présente une face extérieure et une face intérieure et une zone coulissante hémisphérique (2) est réalisée sur la face intérieure pour recevoir un partenaire coulissant sphérique, dans laquelle l'insert (1) est réalisé sous la forme d'un anneau ou sous forme annulaire, et présente circonférentiellement, sur la face extérieure, une surface de serrage (3) conique ou cylindrique au moins dans certaines zones, de sorte que l'insert est relié à l'enveloppe métallique uniquement par l'intermédiaire de la surface de serrage.

2. Coque acétabulaire de remplacement selon la revendication 1, **caractérisée en ce que** l'insert (1) a une largeur (B) de 5 à 20 mm.

3. Coque acétabulaire de remplacement selon la revendication 1 ou 2, **caractérisée en ce que** la face intérieure se raccorde à la face extérieure par l'intermédiaire de rayons.

4. Coque acétabulaire de remplacement selon l'une des revendications 1 à 3, **caractérisée en ce que** la zone coulissante hémisphérique (2) recouvre toute la face intérieure.

5. Coque acétabulaire de remplacement selon l'une quelconque des revendications précédentes, **caractérisée en ce que** la surface de serrage (3) circonférentielle présente des interruptions.

6. Utilisation d'une coque acétabulaire de remplacement selon l'une des revendications 1 à 5 dans l'arthroplastie de la hanche, de l'épaule, de l'orteil ou du genou.
